# EUROPÄISCHE PATENTSCHRIFT

(11) **EP 2 424 465 B1**
(45) Veröffentlichungstag und Bekanntmachung des Hinweises auf die Patenterteilung: **12.08.2015**
(21) Anmeldenummer: 10713118.7
(22) Anmeldetag: 04.03.2010
(51) Int. Cl.: A61F 2/08, A61B 17/04

(54) **IMPLANTIERBARES SYSTEM MIT AUFLÖSUNGSMECHANISMUS BEI DER AUSHEILUNG**
IMPLANTABLE SYSTEM HAVING A DISSOLUTION MECHANISM UPON RECOVERY
SYSTÈME IMPLANTABLE À MÉCANISME DE DÉCOMPOSITION PENDANT LA GUÉRISON

(30) Priorität: 28.04.2009 DE 102009019233; 30.10.2009 DE 102009051367
(43) Veröffentlichungstag der Anmeldung: 07.03.2012
(73) Patentinhaber: Mathys AG Bettlach, 2544 Bettlach (CH)
(72) Erfinder: EGGLI, Stefan, 3007 Bern (CH); DELFOSSE, Daniel, 3303 Jegenstorf (CH); DE CESARIS, Alessandro, 4552 Derendingen (CH); HERWIG, Marianne, 35089 Marburg (DE); KOHL, Sandro, 3006 Bern (CH)
(74) Vertreter: Körfer, Thomas
(86) Internationale Anmeldenummer: PCT/EP2010/001363
(87) Internationale Veröffentlichungsnummer: WO 2010/124760

(56) Entgegenhaltungen:
- WO-A1-97/36557
- DE-U1- 8 914 308
- US-A- 4 187 558
- US-A- 4 942 875
- US-A- 5 507 812

## Beschreibung

Die Erfindung bezieht sich auf einen Auflösungsmechanismus eines Systems, das zur kontrollierten Beanspruchung eines rekonstruierten bzw. renaturierten Bandes in den Knochen implantiert wurde, bei der Ausheilung.

Das menschliche Kniegelenk wird durch das vordere Kreuzband und das hintere Kreuzband im Kniegelenksinnenraum stabilisiert. Bei einem Verdrehtrauma des Kniegelenks, werden diese beiden Bänder sehr oft überlastet, bis es zur Ruptur bzw. zu einem Abreißen kommt. Dabei ist das vordere Kreuzband ca. 9-mal häufiger betroffen als das hintere Kreuzband. Sämtliche konservativen Therapieversuche oder Versuche, das vordere Kreuzband zu nähen, sind mit erheblichen Problemen behaftet. Aus diesem Grund wird bei persistierender Instabilität des verletzten Kniegelenks gemäß dem Stand der Technik das vordere Kreuzband entfernt und die Kniegeleckstabilität durch ein Transplantat aus Sehnenmaterial oder einem synthetischen Band wieder hergestellt. Der Nachteil dieser Methode besteht darin, dass diese Bandstruktur avital ist, keine Sensibilität mehr aufweist und mit zunehmender Dauer wieder an Stabilität verliert.

Die US 6,077,989 A beschreibt ein System gemäß Oberbegriff des Anspruchs 1.

Die US 5,507,812 beschreibt eine modulare Bandprothese, die ein natürliches Band, das die Enden benachbarter Knochen verbindet, ersetzt und deren Beugung ermöglicht. Die Bandprothese umfasst ein erstes und ein zweites Ankerelement, die in den benachbarten Knochen eingebracht sind und eine Kabelanordnung, die die beiden Ankerelemente miteinander verbindet. Das Kabel besteht bevorzugt aus Chrom-Kobalt und ist im zweiten Ankerelement an einem Dämpfungselement gekoppelt. Durch die beschriebene Bandprothese werden zwei Knochen mit einstellbarer Spannung zusammengehalten und können gebeugt werden.

Der Nachteil bei dieser Vorrichtung besteht darin, dass das vordere Kreuzband im menschlichen Kniegelenk durch ein Ersatzband permanent ersetzt wird. Dabei wird das beschädigte natürliche Band endgültig aus dem Kniegelenk entfernt, wobei das künstliche Ersatzband dessen Funktion nur unzureichend übernimmt. Insbesondere geht die Sensibilität komplett verloren, was zu Überlastung führen kann. Daneben unterliegt die künstliche Bandprothese einem Verschleißprozess, was somit nach einem bestimmten Zeitraum zur Instabilität oder gar zu einer erneuten Ruptur führen kann.

Andererseits weist jedes Band des menschlichen Körpers eine weitreichende Selbstheilungstendenz auf. So werden zum heutigen Zeitpunkt fibulotalare Bandrupturen oder AC-Gelenksrupturen praktisch alle konservativ behandelt. Selbst der Riss der großen Achillessehne wird an vielen Zentren mittlerweile konservativ behandelt. Dabei wird das Band rekonstruiert bzw. renaturiert, indem die vorhandenen Bandbüschel eines gerissenen Bandes einander angenähert werden, sodass die Bandbüschel durch die Selbstheilungstendenz wieder zusammenwachsen.

Der Erfindung liegt die Aufgabe zugrunde, ein System für eine temporäre Entlastung eines rekonstruierten, bzw. renaturierten natürlichen vorderen Kreuzbands im menschlichen Kniegelenk bzw. für eine beliebige Bandstruktur eines menschlichen oder tierischen Gelenks anzugeben, die sich in bzw. nach der Ausheilungsphase des natürlichen Bandes wieder selbst abbaut.

Die Aufgabe wird durch das erfindungsgemäße System und dem beschriebenen Auflösungsmechanismus bei der Ausheilung gemäß Anspruch 1 gelöst. In den Unteransprüchen sind vorteilhafte Weiterbildungen des erfindungsgemäßen Systems dargestellt.

Das erfindungsgemäße System für die kontrollierte Beanspruchung, d.h. Stabilisierung und Schutz vor Überlastung eines rekonstruierten, bzw. renaturierten menschlichen oder tierischen Bandes während der Heilungsphase besteht aus einem Verankerungselement zur Implantation in einem ersten Knochen, mindestens einem Verbindungselement und einem Halteelement für das zumindest eine Verbindungselement an einem zweiten Knochen, wobei das Verankerungselement und/oder das Verbindungselement und/oder das Halteelement aus selbstauflösendem, bio-resorbierbarem Material bestehen.

Durch das erfindungsgemäße System wird der Unterschenkel gegenüber dem Oberschenkel dauernd in eine hintere Schublade gezogen. Dadurch werden die zwei gerissenen Faserbündel z.B. des vorderen Kreuzbandes einander auf eine kürzest mögliche Distanz angenähert. Vorteilhafterweise können die beiden Bandstümpfe mit Hilfe des erfindungsgemäßen Systems ohne die verlorengegangene Stabilität wieder in der ursprünglicher Position und Länge zusammenheilen und ihre ursprüngliche Funktion, insbesondere die Stabilisierung des Gelenks wieder vollständig erfüllen.

Vorteilhafterweise bestehen das Verankerungselement und/oder das Verbindungselement und/oder das Haltelement aus selbstauflösendem, bio-resorbierbarem Material. Dieses löst sich mit der Zeit selbst auf, sodass die haltende und stabilisierende Funktion sukzessive auf das natürliche Band übergeht. Somit ist ein kontinuierlicher Übergang der haltenden und stabilisierenden Wirkung vom Ersatzband auf das natürliche Band gegeben. Von enormem Vorteil ist es, dass das Bandimplantat nicht durch eine weitere Operation aus dem Kniegelenk entfernt werden muss. Dies stellt ein Risiko für den Patienten dar und erhöht die Behandlungskosten.

Es ist ebenfalls von Vorteil, dass die Fadenspannung während des Auflösungsprozesses des Verankerungselements und/oder des Halteelements und/oder des Verbindungselements abnimmt. Das natürliche Band wird dadurch unter kontinuierlicher, zunehmender Spannung gehalten, sodass das Wachstum von Bandmaterial angeregt wird. Dies fördert einen gleichmäßigen und raschen Heilungsverlauf.

Es ist ausreichend, wenn zumindest eines der drei Elemente aus bioresorbierbarem Material besteht. Die bevorzugt verwendeten Materialien für die sich nicht auflösenden Elemente sind dann:
- rostfreie Stähle, Ti- oder CoCr-Legierungen, biokompatible Polymere für das Verankerungselement;
- Faden aus Polyethylen, Polyamid oder andern Polymeren für das Verbindungselement und.
- rostfreie Stähle, Ti- oder CoCr-Legierungen, biokompatible Polymere für das Halteelement.

Im Folgenden sind die Elemente so beschrieben, dass sie sich im Körper nach einer bestimmten Dauer auflösen können.

Vorteilhafterweise bestehen das Verankerungselement oder das Befestigungselement aus sich auflösendem Magnesium. Magnesiumschrauben werden zur Verwendung als Knochenschrauben bei medizinischen Anwendungen beschrieben. Die verwendeten Magnesiumlegierungen weisen etwas geringere mechanische Eigenschaften als medizinischer Stahl oder Titan, aber deutlich höhere als bioresorbierbare Polymere auf. Die vollständige Degradation im Körper setzt eine uneingeschränkte Biokompatibilität voraus. Das bedeutet absolute Unschädlichkeit für den Organismus. Dabei muss der Implantatwirkstoff aber durch seine Korrosionsfähigkeit in der Körperumgebung die Voraussetzung zum Abbau des Materials erfüllen. Magnesium als essenzieller Bestandteil des menschlichen Körpers erfüllt alle Voraussetzungen. Zur Einstellung der gewünschten Verweildauer ist das Magnesium bevorzugt oberflächenbehandelt.

Durch das Außengewinde im Verankerungselement kann dieses stabil im ersten Knochen fixiert werden. Das Außengewinde ermöglicht in vorteilhafter Weise eine stufenlose Einsetztiefe, die zur genauen Einstellung der Zugbelastung auf das Verbindungselement nutzbar ist.

Von Vorteil ist es ebenfalls, dass das Befestigungselement mit einer Dämpfungseinrichtung im Verankerungselement gekoppelt ist. Die Dämpfungseinrichtung besteht bevorzugt aus einer Einzelfeder oder einer Doppelfeder, die aus zwei koaxial angeordneten Federn besteht. Die Dämpfungseinrichtung erlaubt eine Beugung des Gelenks und verhindert gleichzeitig eine starke Belastung des sich regenerierenden Bandes bei einer unkontrollierten Bewegung. Die Feder gleicht dabei die übliche dynamische Belastung aus. Bei der Doppelfeder werden Spitzenbelastungen durch die zusätzliche Federwirkung des zweiten Federelements ebenfalls abgefangen.

Vorteilhafterweise ist das Befestigungselement als Kegel ausgeführt und das Verbindungselement zwischen einer konisch zulaufenden Hülse und dem Kegel verklemmt. Das Befestigungselement kann im Weiteren aus mehreren Kegelsegmenten bestehen, wobei nun das Verbindungselement zwischen den Kegelsegmenten eingelegt wird und die Kegelsegmente in eine konisch zulaufende Hülse eingeschoben werden. Das Verbindungselement wird bei der Implantation mit einer gewünschten Vorspannung in distale Richtung gezogen. Der Kegel bzw. die Kegelsegmenten werden noch während dieser Vorspannung in die vorgesehene Hülse eingeschoben und damit auf ihrer Position fixiert. Ein axiales Nachrutschen des Verbindungselements in proximale Richtung wird damit weitestgehend verhindert.

Die Hülse verjüngt sich in Zugrichtung des Verbindungselements. Dies bewirkt in vorteilhafter Weise, dass bei steigender Zugspannung die Kegelsegmente bzw. der Kegel weiter in die Hülse eingepresst werden und die Klemmung sich dadurch verstärkt. Der Klemmdruck wirkt zudem auf die gesamte Länge des Verbindungselements und verhindert eine punktuelle Verletzung und somit das Abreißen des Verbindungselements.

Eine weitere vorteilhafte Variante stellt ein Befestigungselement als Keil dar, der mit seinem spitzen Ende in zwei sich verjüngende ebene Flächen eingeschoben wird, wobei das Verbindungselement um den Keil gelegt ist und zwischen Keil und sich verjüngende ebene Flächen verklemmt ist. Der Klemmdruck wird hier auf einen noch längeren Bereich des Verbindungselements verteilt. Auch hier wird bei zunehmender Zugspannung der Keil weiter in die sich verjüngende ebene Flächen geschoben und somit die Verklemmung verstärkt.

Das Verbindungselement ist vorteilhafterweise aus einem bioresorbierbaren Polymer aufgebaut, bevorzugt aus Poly(glykolsäure), Poly(glykolsäure-co-milchsäure), Poly(glykolsäure-co-DL-milchsäure), Poly(L-milchsäure), Poly(DL-milchsäure), Poly(D-milchsäure), Poly(milchsäure-co-ε-caprolacton), Poly(ε-caprolacton) oder Poly(dioxanon). Verbindungselemente aus Poly(DL-milchsäure) oder Poly(D-milchsäure), beginnen z.B. nach ca. 8 Wochen eine hydrolytische Umwandlung in kürzere Polymerketten. Das Verbindungselement ist nach ca. 3 bis 24 Monaten vollständig aufgelöst. Dieser Zeitraum reicht für die Ausheilung des natürlichen Bandes aus. Durch die Wahl unterschiedlicher Polymere kann die Verweildauer des Fadens im Körper eingestellt und der voraussichtlichen Heilungsdauer von 3 bis 6 Monaten angepasst werden.

Das Halteelement besteht vorteilhafterweise aus bioresorbierbaren Calcium-Phosphat oder Magnesium. Das Halteelement ist knopfförmig ausgebildet und weist am Außenrand diametral gegenüberliegend zwei Führungsöffnungen auf. Während der Implantation kann durch Fäden in den Führungsöffnungen das Halteelement genau positioniert werden. Zur Befestigung des Verbindungselements wird das Ende der Fäden um einen Mittelsteg des knopfförmigen Halteelements geführt und mit dem Verbindungselement selber verspleißt, verschweißt oder geklebt. Das so verbundene Ende des Verbindungselements sorgt für einen festen Zusammenhalt und Zugfestigkeit des Elements.

Vorteilhafterweise wird das beschriebene erfindungsgemäße System zur temporären Entlastung des vorderen Kreuzbandes in ein Kniegelenk eingesetzt. Rupturen des vorderen Kreuzbandes treten häufig als Folge von Distortionen, Sportunfällen oder durch Abnutzungserscheinungen auf. Durch den beschriebenen Auflösungsmechanismus des Implantats kann das permanente Verbleiben des Systems im Kniegelenk, das zu Reizungen etc. führen kann, verhindert werden. Andererseits wird dem Patienten eine belastende Operation zur Entnahme des implantierten Systems erspart.

Ausführungsbeispiele des erfindungsgemäßen Systems bzw. Teilkomponenten davon sind in den Zeichnungen beispielhaft dargestellt und werden anhand der nachfolgenden Beschreibung näher erläutert. Es zeigen:
- Fig.1: ein erfindungsgemäßes System eingesetzt in ein Kniegelenk als Ersatz für das vordere Kreuzband in schematischer Darstellung;
- Fig.2: einen Schnitt durch ein erstes Ausführungsbeispiel eines erfindungsgemäßen Verankerungselements mit einem Kegel als Befestigungselement;
- Fig. 3: einen Schnitt durch ein zweites Ausführungsbeispiel eines erfindungsgemäßen Verankerungselements mit ein Kegelsegmenten als Befestigungselement;
- Fig. 4: einen Schnitt durch ein drittes Ausführungsbeispiel eines erfindungsgemäßen Verankerungselements mit einer zuziehenden Seilklemme als Befestigungselement;
- Fig. 5: einen Schnitt durch ein viertes Ausführungsbeispiel eines erfindungsgemäßen Verankerungselements mit einer Presshülse als Befestigungselement;
- Fig. 6: einen Schnitt durch ein fünftes Ausführungsbeispiel eines erfindungsgemäßen Verankerungselements mit einem Keil als Befestigungselement und
- Fig. 7: ein erfindungsgemäßes Halteelement in perspektivischer Ansicht.

Einander entsprechende Teile sind in allen Figuren mit den gleichen Bezugszeichen versehen.

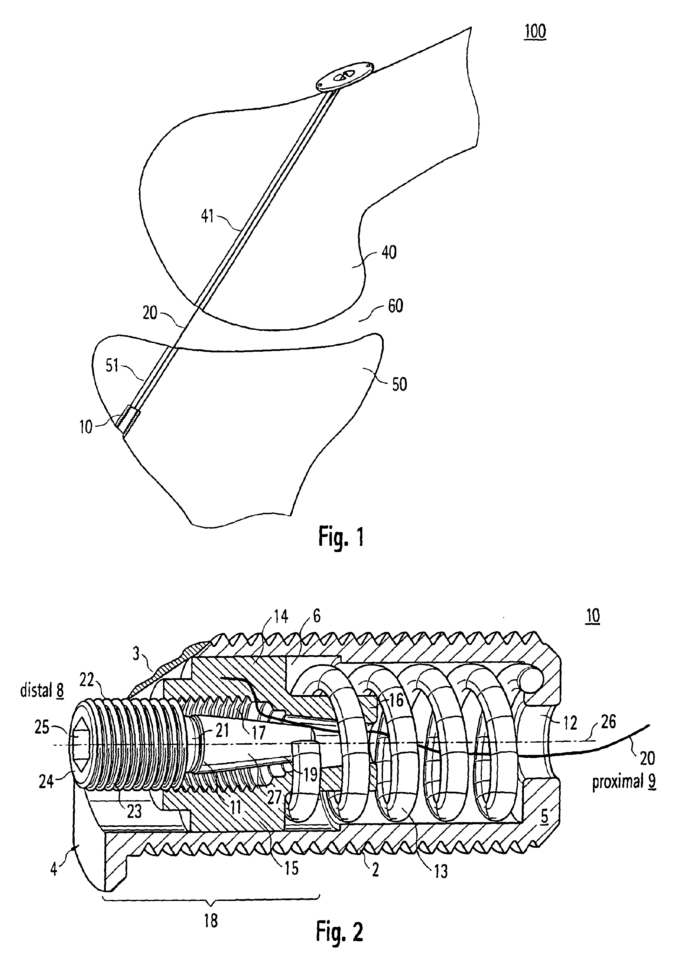

Fig. 1 zeigt das erfindungsgemäße System 100 eingesetzt in ein gebeugtes menschliches Kniegelenk. Das Verankerungselement 10 ist ventral im proximalen Bereich des Tibiaknochens 50 eingeschraubt, an den sich ein erster Knochentunnel 51 anschließt, der zum Gelenkinnenraum 60 führt. Durch das angrenzende distale Ende des Femur-Knochens 40 ist ein schmaler zweiter Knochentunnel 41 gebohrt. Das Verbindungselement 20 ist an einem Halteelement 30, das als sogenannter "Endo-Button" ausgebildet ist, befestigt. Das Halteelement stützt sich dabei an der Außenfläche des Femurs 40 ab. Das Verbindungselement führt durch den zweiten Knochentunnel 41 über den Gelenkinnenraum 60 und den ersten Knochentunnel 51 zum Verankerungselement 10 und wird von diesem fixiert.

Die Lage der Komponenten des implantierten Systems und der Knochentunnel ist so gewählt, dass das Verbindungselement bei einem ca. 90° gebeugten Knie eine Gerade aufspannt. Das Verbindungselement ist in Länge und Vorspannung so vom Operateur eingestellt, dass in der Heilungsphase keine oder nur minimale Zugbelastung auf das rekonstruierte, heilende menschliche Band lastet. Mit fortschreitender Heilungsphase lösen sich das Verankerungselement und/oder das darin fixierte Verbindungselement und/oder das Verbindungselement selbst und/oder auch das Halteelement auf und übertragen damit mehr und mehr der natürlichen Kräfte auf das heilende menschliche Band.

Eines oder mehrere dieser Elemente bestehen aus bio-resorbierbarem, sich selbstauflösendem Material. Das sich auflösende Verankerungselement besteht aus Magnesium. Das bio-resorbierbares Halteelement besteht ebenfalls aus Magnesium oder aus Calcium-Phosphat. Resorbierbare Verbindungselemente bestehen Vorteilhafterweise aus Polymeren z.B. Polylactiden, Polyglycolsäure, Poly-ε-capolactole oder auch Polydiohoxanon.

Mit dem Einsetzen des Auflösungsvorgangs nimmt die Spannung im Verbindungselement ab und das natürliche Band wird zunehmend belastet. Dies regt verstärkt die Regeneration des natürlichen Bandes an und fördert somit den Heilungsprozess und die Heilungsgeschwindigkeit. Nach dem vollständigen Auflösen des Verbindungselements bzw. des Verankerungselements bzw. des Halteelements übernimmt das natürliche Band wieder vollständig seine natürliche Funktion.

Im Gegensatz zu herkömmlichen Implantaten verbleibt nicht das ganze Bandimplantat im Körper und stört somit den natürlichen Bewegungsablauf nicht, so dass es nicht operativ entfernt werden muss. Die verbleibenden Knochentunnel bzw. die Bohrung für das Verankerungselement wachsen im Laufe der Zeit mit neuem Knochengewebe zu.

Fig. 2 zeigt eine seitlich geschnittene Darstellung des erfindungsgemäßen Verankerungselements 10. Dies umfasst einen zylinderförmigen Außenkörper 3, der mit einem Außengewinde 2 versehen ist und damit in einen Knochen 50 eingeschraubt wird. Dabei verankert sich der zylinderförmige Außenkörper 3 selbst im Knochengewebe. Der zylinderförmige Außenkörper 3 weist eine Stirnseite 4 und einen Boden 5 auf, wobei die Stirnseite 4 nach dem Einbringen in den Knochen 50 zur Knochenoberfläche hin orientiert ist. Im Inneren 6 des erfindungsgemäßen Verankerungselements 10 bzw. des zylinderförmigen Außenkörpers 3 ist eine Dämpfungseinrichtung zwischen einem distalen Ende 8 und einem proximalen Ende 9 untergebracht. Das Befestigungselement 19 ist bevorzugt als Kegel 27 ausgeführt und fixiert ein Verbindungselement 20, welches größtenteils innerhalb einer Dämpfungseinrichtung parallel zu ihrer Längsachse 26 verläuft und am proximalen Ende 9 der Vorrichtung 10 durch eine Aussparung 12 am Boden 5 aus dem zylinderförmigen Außenkörper 3 herausgeführt ist.

Die Dämpfungseinrichtung ist mit einer Spiralfeder 13 ausgestattet, welche mit einem vom Operateur veränderbaren Druck beaufschlagt ist und an ihrem proximalen Ende 9 auf dem Boden 5 des zylinderförmigen Außenkörpers 3 aufliegt. Ferner ist innerhalb der Dämpfungsvorrichtung eine Hülse 14 angeordnet, welche einen Flansch 15 mit einem Innengewinde 17 aufweist, auf welchem die Spiralfeder 13 mit ihrem distalen Ende aufliegt. Das proximale Ende 16 der Hülse 14 ist in die Spiralfeder 13 hineingeschoben, sodass diese die Hülse 14 im Bereich der distalen Hälfte 18 des zylinderförmigen Außenkörpers 3 umgibt. Das als Kegel 27 ausgeformte Befestigungselement 19 ist mit seinem distalen Ende 11 an einem Schraubenfortsatz 20 einer Schraube 22 befestigt, wobei die Schraube 22 für ein kontrolliertes Abwickeln des Fadens 11 auf dem Kegel 27 vorgesehen ist, wodurch sich die Spannung auf das Verbindungselement 20 erhöht. Da die Schraube 22 im Bereich des Schraubenkopfes 24 eine Aussparung 25 in Form eines Mehrecks aufweist, ist sie mit ihrem Außengewinde 23 in das Innengewinde 17 des Flansches 15 einschraubbar.

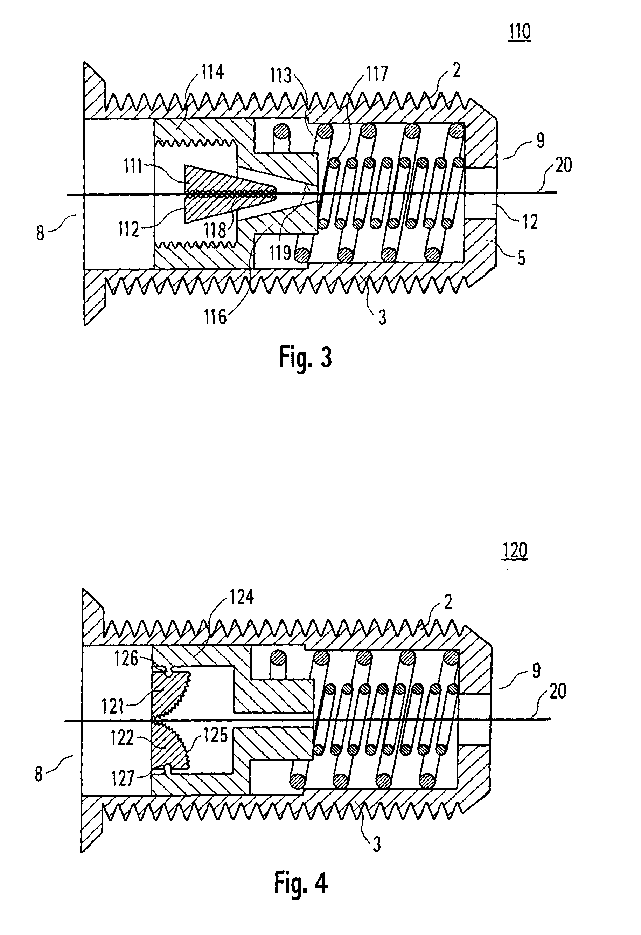

Ein zweites Ausführungsbeispiel eines erfindungsgemäßen Verankerungselements 110 ist in Fig. 3 dargestellt. Das zylinderförmige Außengehäuse 3 mit seinem Außengewinde 2 ist wiederum in dem Knochen eingedreht. Die Hülse 114 ragt mit ihrem proximalen Ende 116 wiederum in eine Spiralfeder 113 hinein. Eine zweite Spiralfeder 117 liegt koaxial in der ersten Spiralfeder 113 und wird von dieser vollständig umfasst. Die zweite Spiralfeder 117 stützt sich einerseits an dem Boden 5 des Außengehäuses 3 sowie am proximalen Ende der Hülse 116 ab. Durch diese Doppelfederanordnung kann eine höhere Federkonstante erreicht werden und somit stärkere Zugspannungen, z.B. durch unerwartete Bewegungen des Knies, abgefedert werden, ohne zusätzlichen Raum für eine größere und damit stärkere Feder zu benötigen. Mit einer verkürzten zweiten Feder 117 kann eine stufenweise Erhöhung der Federkonstanten erreicht werden.

Das Befestigungselement wird durch mindestens zwei Kegelsektoren 111, 112 gebildet. Die Innenfläche 119 zumindest des proximalen Endes der Hülse 116 läuft konisch zu und entspricht in ihrer Neigung dem Befestigungselementen 111 und 112. Das Verbindungselement 20 wird durch die Aussparung 12 in den Innenraum des Außengehäuses 3 eingeführt, durch das proximale Ende der Hülse 116 geführt und zwischen die Kegelsektoren 111 und 112 eingelegt. Die Innenfläche 118 der Kegelsegmente 112 und 113 können gezackt ausgeführt sein, um einen besseren Halt am Verbindungselement 20 zu gewährleisten. Die Kegelsektoren 111, 112 werden in das proximalen Endes der Hülse 116 eingeschoben. Somit wird das Verbindungselement 20 fixiert. Je stärker die Zugkraft in proximale Richtung auf das Verbindungselement 20 wirkt, desto stärker werden die Kegelsektoren 111, 112 verkeilt und die Befestigung verstärkt. Optional kann das Verbindungselement 20 durch eine axial ausgehöhlte Schraube 22, siehe Fig.2, geführt werden und mittels Einschrauben der Schraube 22 in das Innengewinde 23 die Kegelsegmente in das proximale Ende der Hülse 116 eingeschoben werden.

Fig. 4 zeigt ein Verankerungselement 120, das in gleicher Weise wie das Verankerungselement 110 aufgebaut ist, in dessen Hülse 124 jedoch statt den Kegelsegmenten eine zuziehende Seilklemme 124 angebracht ist. Die Seilklemme wird aus zwei drehbar gelagerten Backen 121 und 122 gebildet, deren Oberfläche 125 gezackt ausgeformt ist. Zum Einlegen des Verbindungselements 20 wird das Verbindungselements 20 zwischen den Backen 121 und 122 zum distalen Ende 8 des Außengehäuses 3 hin eingeschoben. Dabei drehen sich die ineinander greifenden Backen 121, 122 um ihre Achsen 126, 127 in distale Richtung und öffnen durch ihren sich verkleinenden Radius. Beim Lösen der Spannung schnappen die beiden Backen 121 und 122 in proximale Richtung 9 zurück und Verklemmen das Verbindungselement 20. Bei einer Zugkraft in proximale Richtung wird die Verklemmung fester gezogen und somit verstärkt.

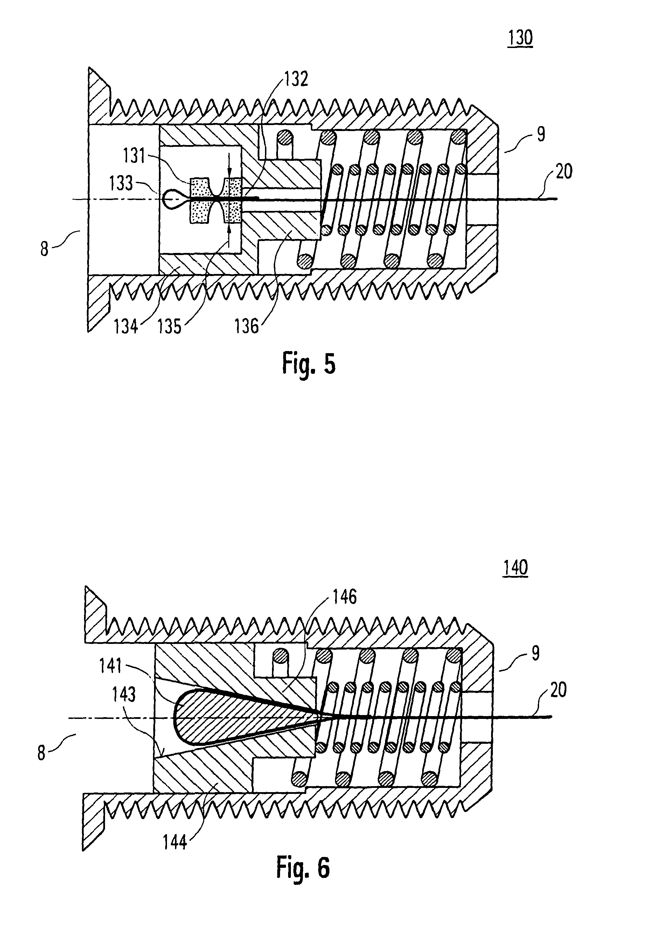

Fig. 5 zeigt eine weitere Ausführungsform 130 des erfindungsgemäßen Verankerungselements. Das Befestigungselement 20 wird durch das proximale Ende 136 der Hülse 134 geführt. Das Ende des Verbindungselements 20 wird zu einer Schlaufe 133 geformt und das Ende in einer Presshülse 131 mit dem Verbindungselement verklemmt. Die Presshülse 131 hat dabei einen Durchmesser 135 in der Größe, dass ein Durchrutschen durch das proximale Ende der Hülse 116 unmöglich ist.

Fig. 6 zeigt ein fünftes Ausführungsbeispiel 140 eines erfindungsgemäßen Verankerungselements 10 mit einer Keilverklemmung. Die Hülse des Verankerungselements 144 bildet auf der Innenseite als spitz zulaufende ebene Flächen 142, 143 aus. Das Verbindungselement 20 wird durch das proximale Ende der Hülse 146 hindurchgeführt um den Keil 141 und zurück in die proximale Hälfte 9 des Außengehäuses zurückgeführt. Wirkt eine Zugkraft in proximale Richtung auf das Verbindungselement 20, wird der Keil 141 in die spitz zulaufenden Innenflächen 142, 143 der Hülse eingepresst und das Verbindungselement 20 eingeklemmt. Hier wirkt wiederum eine proximale Zugkraft festigend auf die Verklemmung.

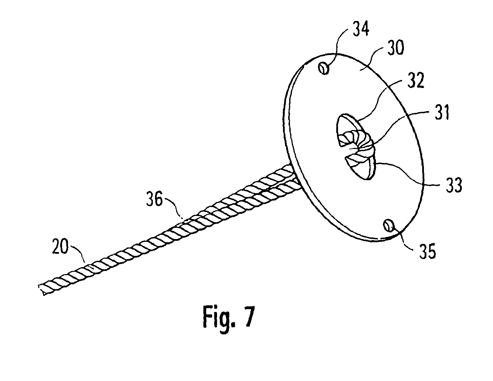

Fig. 7 zeigt das Halteelement 30 zusammen mit dem an ihm fixierten Verbindungselements 20. Das Halteelement 30 ist knopfförmig geformt und weist zwei Öffnungen 32, 33 und einen dazwischen liegenden Mittelsteg 31 auf. Das Verbindungselement 20 wird durch die Öffnung 33 hindurch geführt und über die Öffnung 32 wieder zurück gelegt. Das Ende 36 des Verbindungselements 20 ist mit dem Verbindungselement 20, das bevorzugt als aus mehreren Einzelfasern geflochtener Faden ausgebildet ist, verspleißt. Bevorzugt ist das Halteelement 30 mit verspleißten Verbindungselement vormontiert und wird als eine Komponente implantiert. Um das Halteelement 30 optimal an der Oberfläche des Femur-Knochens 40 ausrichten zu können, sind zwei Führungsöffnungen 34, 35 am Außenrand diametral gegenüber angebracht. Während der Implantation werden dünne Fäden an diesen Führungsöffnungen 34, 35 angebracht und das Halteelement 30 damit positioniert und ausgerichtet.

Alle beschriebenen und/oder gezeichneten Merkmale können im Rahmen der Erfindung vorteilhaft miteinander kombiniert werden. Die Erfindung ist nicht auf die Ausführungsbeispiele beschränkt. Beispielsweise können auch andere Klemmmechanismen oder andere Materialien eingesetzt werden.

## Patentansprüche

1. System für eine kontrollierte Beanspruchung eines rekonstruierten oder renaturierten Bandes eines menschlichen oder tierischen Körpers während der Heilungsphase, welches ein Verankerungselement (10) zur Implantation in einem ersten Knochen (50), mindestens ein Verbindungselement (20) und ein Halteelement (30) für das zumindest eine Verbindungselement (20) an einem zweiten Knochen (40), umfasst, wobei das Verankerungselement (10) und/oder das Verbindungselement (20) und das Halteelement (30) aus selbstauflösendem, bio-resorbierbarem Material bestehen und das Halteelement (30) aus bio-resorbierbarem Calcium-Phosphat oder Magnesium besteht,
**dadurch gekennzeichnet,**
**dass** das Halteelement (30) knopfförmig ausgebildet ist und ein Verbindungselementende (36) um einen Mittelsteg (31) des knopfförmigen Halteelements (30) geführt und mit dem Verbindungselement (20) verspleißt, verschweißt oder geklebt ist. Phosphat oder Magnesium besteht und knopfförmig ausgebildet ist und ein Verbindungselementende (36) um geführt und mit dem Verbindungselement (20) verspleißt, verschweißt oder geklebt ist.

2. System nach Anspruch 1,
**dadurch gekennzeichnet,**
**dass** die Spannung in dem Verbindungselement (20) während des Auflösungsprozesses des Verankerungselements (10) und/oder des Verbindungselements (20) und des Halteelements (30) abnimmt.

3. System nach Anspruch 1 oder 2,
**dadurch gekennzeichnet,**
**dass** das Verankerungselement (10) ein Außengewinde (2) aufweist und mit diesem im ersten Knochen (50) fixiert ist.

4. System nach einem der Ansprüche 1 bis 3,
**dadurch gekennzeichnet,**
**dass** das Verbindungselement (20) an einem Befestigungselement (19) im Verankerungselement (10) fixiert ist.

5. System nach Anspruch 4,
**dadurch gekennzeichnet,**
**dass** das Verankerungs- (10) oder das Befestigungselement (19) aus sich auflösendem Magnesium oder Calcium-Phosphat besteht.

6. System nach Anspruch 4 oder 5,
**dadurch gekennzeichnet,**
**dass** das Befestigungselement (19) mit einer Dämpfungseinrichtung im Verankerungselement (10) gekoppelt ist.

7. System nach Anspruch 6,
**dadurch gekennzeichnet,**
**dass** die Dämpfungseinrichtung eine Einzelfelder oder eine Doppelfeder, bestehend aus zwei koaxialen Federn (113, 117), umfasst.

8. System nach einem der Ansprüche 4 bis 7,
**dadurch gekennzeichnet,**
**dass** das Befestigungselement (19) als Kegel (27) ausgeführt ist und das Verbindungselement zwischen einer konisch zulaufenden Hülse (14, 16) und dem Kegel (27) verklemmt ist.

9. System nach einem der Ansprüche 4 bis 8,
**dadurch gekennzeichnet,**
**dass** das Befestigungselement (19) aus mehreren Kegelsegmenten (111, 112) besteht, das Verbindungselement (20) zwischen Kegelsegmente (111, 112) eingelegt und in eine konisch zulaufende Hülse (114,116) eingeschoben ist.

10. System nach Anspruch 8 oder 9,
**dadurch gekennzeichnet,**
**dass** sich die Hülse (14, 16, 114, 116) in proximale Richtung (9) verjüngt.

11. System nach einem der Ansprüche 4 bis 7,
**dadurch gekennzeichnet,**
**dass** das Befestigungselement (19) als Presshülse (131) ausgeführt ist, die das Ende (132) einer Verbindungselementschlaufe (133) mit dem Verbindungselement (20) verklemmt.

12. System nach einem der Ansprüche 4 bis 7,
**dadurch gekennzeichnet,**
**dass** das Befestigungselement (19) als Keil(141) ausgeführt ist, der mit seinem spitzen Ende in zwei sich verjüngende ebene Flächen (142, 143) eingeschoben ist, wobei das Verbindungselement (20) um den Keil (141) gelegt ist und zwischen Keil (141) und den ebenen Flächen (142, 143) verklemmt ist.

13. System nach einem der Ansprüche 1 bis 12,
**dadurch gekennzeichnet,**
**dass** das Verbindungselement (20) aus bio-resorbierbarem Polymer, bevorzugt aus Poly(glykolsäure), Poly(glykolsäure-co-milchsäure), Poly(glykolsäure-co-DL-milchsäure), Poly(L-milchsäure), Poly(DL-milchsäure), Poly(D-milchsäure), Poly(milchsäure-co-ε-caprolacton), Poly(ε-caprolacton) oder Poly(dioxanon), besteht.

14. System nach einem der Ansprüche 1 bis 13,
**dadurch gekennzeichnet,**
**dass** das Verbindungselement (20) als ein Faden, insbesondere als ein aus mehreren Einzelfasern geflochtener Faden, ausgebildet ist.

15. System nach einem der Ansprüche 1 bis 14,
**dadurch gekennzeichnet,**
**dass** zwei Führungsöffnungen (34, 35) diametral gegenüberliegend am Außenrand des Halteelements(30) eingebracht sind.

16. System nach Anspruch 1 bis 15,
**dadurch gekennzeichnet,**
**dass** das System zur temporären Entlastung des vorderen Kreuzbandes in ein Kniegelenk eingesetzt ist.

## Claims

1. System for controlled loading of a reconstructed or restored ligament of a human or animal body during the recovery phase, which comprises an anchoring element (10) for implantation in a first bone (50), at least one connecting element (20) and a holding element (30) for the at least one connecting element (20) on a second bone (40),
wherein the anchoring element (10) and/or the connecting element (20) and the holding element (30) are made of self-dissolving bioresorbable material and the holding element (30) is made of bioresorbable calcium phosphate or magnesium, **characterised in that** the holding element (30) is button-shaped and one end (36) of the connecting element is passed around a central member (31) of the button-shaped holding element (30) and spliced, welded or glued to the connecting element (20).

2. System according to claim 1,
**characterised in that** the tension in the connecting element (20) reduces during the dissolving process of the anchoring element (10) and/or of the connecting element (20) and the holding element (30).

3. System according to claim 1 or 2,
**characterised in that** the anchoring element (10) exhibits an external thread (2) and is fixed in the first bone (50) with this.

4. System according to one of claims 1 to 3,
**characterised in that** the connecting element (20) is fixed to a fastening element (19) in the anchoring element (10).

5. System according to claim 4,
**characterised in that** the anchoring element (10) or the fastening element (19) is made of self-dissolving magnesium or calcium phosphate.

6. System according to claim 4 or 5,
**characterised in that** the fastening element (19) is coupled with a damping arrangement in the anchoring element (10).

7. System according to claim 6,
**characterised in that** the damping arrangement comprises a single spring or a double spring, consisting of two coaxial springs (113, 117).

8. System according to one of claims 4 to 7,
**characterised in that** the fastening element (19) is embodied as a cone (27) and the connecting element is clamped between a conically tapering sleeve (14, 16) and the cone (27).

9. System according to one of claims 4 to 8,
**characterised in that** the fastening element (19) consists of a plurality of cone segments (111, 112), the connecting element (20) is laid between cone segments (111, 112) and slid into a conically tapering sleeve (114, 116).

10. System according to claim 8 or 9,
**characterised in that** the sleeve (14, 16, 114, 116) tapers in the proximal direction (9).

11. System according to one of claims 4 to 7,
**characterised in that** the fastening element (19) is embodied as a compression sleeve (131) which clamps the end (132) of a loop (133) of the connecting element with the connecting element (20).

12. System according to one of claims 4 to 7,
**characterised in that** the fastening element (19) is embodied as a wedge (141) which is slid with its pointed end into two tapering flat surfaces (142, 143), wherein the
connecting element (20) is laid around the wedge (141) and clamped between the wedge (141) and the flat surfaces (142, 143).

13. System according to one of claims 1 to 12,
**characterised in that** the connecting element (20) is made of bioresorbable polymer, preferably of poly(glycolic acid), poly(glycolic acid-co-lactic acid), poly(glycolic acid-co-DL-lactic acid), poly(L-lactic acid), poly(DL-lactic acid), poly(D-lactic acid), poly(lactic acid-co-ε-caprolactone), poly(ε-caprolactone) or poly(dioxanone).

14. System according to one of claims 1 to 13,
**characterised in that** the connecting element (20) is embodied as a thread, in particular a thread braided from a plurality of individual fibres.

15. System according to one of claims 1 to 14,
**characterised in that** two guide openings (34, 35) are formed lying diametrically opposite one another at the outer edge of the holding element (30).

16. System according to claims 1 to 15,
**characterised in that** the system is inserted in a knee joint for temporary relief of the anterior cruciate ligament.

## Revendications

1. Système destiné à une sollicitation contrôlée d'un ligament reconstruit ou renaturé d'un corps humain ou animal, pendant la phase de guérison, qui comprend un élément d'ancrage (10) destiné à être implanté dans un premier os (50), au moins un élément de liaison (20) et un élément de retenue (30) pour ledit au moins un élément de liaison (20) sur un deuxième os (40), système dans lequel l'élément d'ancrage (10) et/ou l'élément de liaison (20) et l'élément de retenue (30) sont réalisés en un matériau dégradable, bio-résorbable, et l'élément de retenue (30) est réalisé en phosphate de calcium bio-résorbable ou magnésium bio-résorbable,
**caractérisé**
**en ce que** l'élément de retenue (30) est d'une configuration en forme de bouton, et une extrémité d'élément de liaison (36) est menée autour d'une nervure centrale (31) de l'élément de retenue (30) en forme de bouton, et est liée à l'élément de liaison (20) par une épissure, par soudage ou par collage.

2. Système selon la revendication 1,
**caractérisé**
**en ce que** la tension dans l'élément de liaison (20) diminue pendant le processus de résorption de l'élément d'ancrage (10) et/ou de l'élément de liaison (20) et de l'élément de retenue (30).

3. Système selon la revendication 1 ou la revendication 2,
**caractérisé**
**en ce que** l'élément d'ancrage (10) présente un filetage extérieur (2) et est fixé par celui-ci dans ledit premier os (50).

4. Système selon l'une des revendications 1 à 3,
**caractérisé**
**en ce que** l'élément de liaison (20) est fixé à un élément de fixation (19) dans l'élément d'ancrage (10).

5. Système selon la revendication 4,
**caractérisé**
**en ce que** l'élément d'ancrage (10) ou l'élément de fixation (19) est réalisé en magnésium ou phosphate de calcium dégradables ou résorbables.

6. Système selon la revendication 4 ou la revendication 5,
**caractérisé**
**en ce que** l'élément de fixation (19) est couplé à un dispositif d'amortissement dans l'élément d'ancrage (10).

7. Système selon la revendication 6,
**caractérisé**
**en ce que** le dispositif d'amortissement comprend un ressort individuel ou un ressort double constitué de deux ressorts coaxiaux (113, 117).

8. Système selon l'une des revendications 4 à 7,
**caractérisé**
**en ce que** l'élément de fixation (19) est réalisé sous forme de cône (27), et l'élément de liaison est serré entre le cône (27) et une douille (14, 16) se rétrécissant de manière conique.

9. Système selon l'une des revendications 4 à 8,
**caractérisé**
**en ce que** l'élément de fixation (19) est constitué de plusieurs segments de cône (111, 112), et l'élément de liaison (20) est placé entre des segments de cône (111, 112) et inséré dans une douille (114, 116) se rétrécissant de manière conique.

10. Système selon la revendication 8 ou la revendication 9,
**caractérisé**
**en ce que** la douille (14, 16, 114, 116) se rétrécit en direction proximale (9).

11. Système selon l'une des revendications 4 à 7,
**caractérisé**
**en ce que** l'élément de fixation (19) est réalisé en tant que douille de compression ou de sertissage (131), qui serre l'extrémité (132) d'une boucle d'élément de liaison (133) avec l'élément de liaison (20).

12. Système selon l'une des revendications 4 à 7,
**caractérisé**
**en ce que** l'élément de fixation (19) est réalisé en tant que coin (141) qui est inséré, avec son extrémité en pointe, dans deux surfaces planes (142, 143) agencées en formant un rétrécissement, l'élément de liaison (20) étant placé autour du coin (141) et serré entre le coin (141) et les surfaces planes (142, 143).

13. Système selon l'une des revendications 1 à 12,
**caractérisé**
**en ce que** l'élément de liaison (20) est réalisé en un polymère bio-résorbable, de préférence en poly(acide glycolique), poly(acide glycolique-co-acide lactique), poly(acide glycolique-co-DL-acide lactique), poly(L-acide lactique), poly(DL-acide lactique), poly(D-acide lactique), poly(acide lactique-co-ε-caprolactone), poly(ε-caprolactone) ou poly(dioxanone).

14. Système selon l'une des revendications 1 à 13,
**caractérisé**
**en ce que** l'élément de liaison (20) est réalisé sous la forme d'un fil, notamment sous la forme d'un fil tressé à partir de plusieurs fibres individuelles.

15. Système selon l'une des revendications 1 à 14,
**caractérisé**
**en ce que** deux ouvertures de guidage (34, 35) sont réalisées de manière diamétralement opposée dans la bordure extérieure de l'élément de retenue (30).

16. Système selon les revendications 1 à 15,
**caractérisé**
**en ce que** le système est implanté dans une articulation de genou pour décharger temporairement le ligament croisé antérieur.
